# EUROPEAN PATENT APPLICATION

(11) **EP 1 614 421 A2**
(43) Date of publication of application: **11.01.2006**
(21) Application number: 05108474.7
(22) Date of filing: 06.02.1998
(51) Int. Cl.: A61K 31/4545, A61K 9/20, A61P 37/08

(54) **Lactose-free, non-hygroscopic and anhydrous pharmaceutical compositions of descarboethoxyloratadine**

(30) Priority: 07.02.1997 US 37325 P; 30.04.1997 US 45184 P; 21.07.1997 US 53050 P
(62) Divisional of application: 98904980.4
(71) Applicant: SEPRACOR, INC., Marlborough, MA 01752 (US)
(72) Inventor: Redmon, Martin P, 01752, Malborough (US); Butler, Hal, T, 01752, Malborough (US); Wald, Stephen A, 01776, Sudbury (US); Rubin, Paul D, 01776, Sudbury (US)
(74) Representative: Killin, Stephen James

(57) **Abstract**

Stable pharmaceutical compositions of descarboethoxyloratadine (DCL), a metabolic derivative of loratadine, for the treatment of allergic rhinitis and other histamine-induced disorders are disclosed. The compositions are formulated to avoid the incompatibility between DCL and reactive excipients such as lactose and other mono- and di-saccharides. Disclosed compositions include lactose-free, non-hygroscopic and anhydrous stable pharmaceutical compositions of DCL.

## Description

This invention relates to pharmaceutical compositions of 8-chloro-6,11-dihydro-11-(4-piperidylidene)-5H-benzo[5,6]cyclohepta[1,2-b]pyridine, known as descarboethoxyloratadine (DCL).

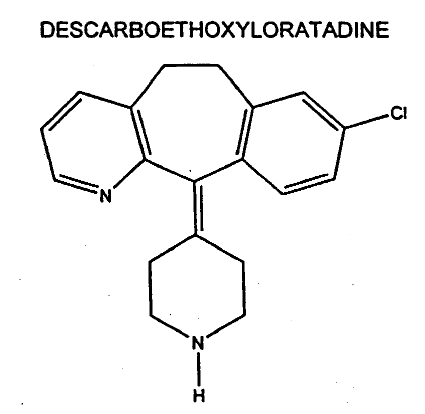

DCL is a metabolic derivative of loratadine, an H-1 histamine receptor antagonist. The H-1 histamine receptors mediate the response Antagonized by conventional antihistamines. Loratadine has been shown to be comparable in antihistaminic activity to terfenadine and astemizole, and to be, on a milligram by milligram basis, four times more potent than terfenadine in the inhibition of allergic bronchospasm.

Loratadine has also been shown to be effective in treating numerous disorders, including colds, chronic urticaria, seasonal allergic rhinitis and seasonal and perennial rhinitis. Due to its antihistaminic activity, loratadine may also be useful for the treatment of allergic asthma, diabetic retinopathy and other small vessel disorders associated with diabetes mellitus.

The administration of antihistamines is frequently associated with adverse side-effects, which include, for example, sedation, headache, dry mouth, constipation or diarrhea, weight gain and gastrointestinal distress. Loratadine belongs to a class of antihistamines referred to as non-sedating antihistamines. This class also includes two other well known antihistamines, terfenadine and astemizolc. In comparison to terfenadine, it has been shown that loratadine causes significantly less sedation than terfenadine and that the incidence of fatigue, headache and nausea associated with loratadine is comparable to that seen with terfenadine.

As a disadvantage to the non-sedating antihistamines, compounds in this class have been reported to cause other severe electrophysicologic side-effects. These adverse side-effects include, but are not limited to, ventricular fibrillation and cardiac arrhythmias, such as ventricular tachyarrhythmias or torsades de pointes. Several of these serious cardiovascular side-effects have been reported in "healthy" patients who received terfenadine concurrently with either ketoconazole or erythromycin. Arrhythmias have also been observed with the concomitant administration of astemizole and erythromycin, and astemizole with erythromycin and ketoconazole. Additionally, it is known that each of ketoconazole, itraconazole and erythromycin interfere with cytochrome P450 and thereby inhibit the metabolism of non-sedating antihistamines such as terfenadine and astemizole. Thus, a strong potential also exists for an adverse interaction between these inhibitors of cytochrome P450 and loratadine. Therefore, due to the similarity in pharmacological activity of loratadine, terfenadine and astemizole, it is also cautioned to avoid the concurrent administration of loratadine with either ketoconazole, itraconazole or a macrolide antibiotic, such as erythromycin.

A further drawback to both astemizole and loratadine is that the administration of each drug has been associated with the growth of both melanoma and fibrosarcoma tumors. The dosage of loratadine being maintained during this observation was 10 mg/day.

Although loratadine is well absorbed, it is extensively metabolized, yielding pharmacologically active descarboethoxyloratadine (DCL) as its main metabolite. Significantly, U.S. Patent 5,595,997, issued January 21, 1997, discloses that DCL, while providing effective, non-sedating antihistaminic therapy, also avoids the many, often severe, adverse side-effects commonly associated with the administration of both antihistamines in general and with other non-sedating antihistamines, such as loratadine, terfenadine and astemizole, in particular.

Importantly, it has been shown that DCL is five to seven times less active in tumor promotion than loratadine and that DCL is at least about twenty times more potent at the histamine receptor when compared to loratadine. Thus, pharmaceutical compositions containing DCL, or a pharmaceutically acceptable salt thereof, as the active ingredient are particularly desirable.

EP 0,396,404 discloses a sustained release pharmaceutical composition in the form of a coated tablet having a core comprising ibuprofen and pseudoephedrine and a swellable hydrophilic polymer, and an outer coating comprising loratadine or DCL and a hydrophilic polymer. On contact with gastric or aqueous media, the outer coating of the tablet dissolves rapidly to release loratadine or DCL, whilst the core is hydrated so as to provide sustained release of ibuprofen and pseudoephedrine.

US 4,659,716 and related International application WO 85/03707 disclose various 7- and/or 8-(halo or trifluoromethyl)-substituted-6,11-dihydro-11-(4-piperidylidene)-5H-benzo[5,6]cyclohepta[1,2-b]pyridines, methods for preparing such compounds and their use as antihistaminic agents. Example A of both documents discloses a tablet formulation that includes an active compound, such as DCL, and lactose. Example B of both documents discloses a capsule formulation that includes an active compound, such as DCL, and lactose in the form of a dry powder blend.

WO 96/16641 discloses pharmaceutical compositions for systemic transdermal administration containing descarboethoxyloratadine (DCL). Example 2 discloses a pharmaceutical composition that comprises DCL and Duro-Tak 1753, which is applied to a laminar structure to form a transdermal patch.

Accordingly, in a first aspect of the invention, there is provided a pharmaceutical composition for the treatment of histamine-induced disorders, comprising 1% to 50% by weight of descarboethoxyloratadine, or a pharmaceutically acceptable salt thereof, and 99% to 50% by weight of a pharmaceutically acceptable inert carrier, wherein the composition is substantially free of reactive excipients and is in tablet form comprising descarboethoxyloratadine, or a pharmaceutically acceptable salt thereof, that has been blended or granulated with a pharmaceutically acceptable inert carrier.

In a second aspect of the invention, there is provided a pharmaceutical composition for the treatment of histamine-induced disorders, comprising descarboethoxyloratadine, or a pharmaceutically acceptable salt thereof, and a pharmaceutically acceptable inert carrier, wherein the composition is substantially free of reactive excipients and is in instant release tablet form comprising descarboethoxyloratadine, or a pharmaceutically acceptable salt thereof, that has been blended or granulated with a pharmaceutically acceptable inert carrier, said tablet being adapted to disintegrate rapidly on contact with water.

In a third aspect of the invention, there is provided a pharmaceutical composition for the treatment of histamine-induced disorders, comprising descarboethoxyloratadine, or a pharmaceutically acceptable salt thereof, and a pharmaceutically acceptable inert carrier, wherein the composition is substantially free of reactive excipients and is in capsule form comprising a powder blend of descarboethoxyloratadine, or a pharmaceutically acceptable salt thereof, in admixture with a pharmaceutically acceptable inert carrier.

In a fourth aspect of the invention, there is provided the use of a descarboethoxyloratadine, or a pharmaceutically acceptable salt thereof for the preparation of a medicament according to the invention in any of its previous aspects for use in a method of treating cough, cold, cold-like and flu symptoms and the discomfort, headache, pain, fever and general malaise associated therewith, a method of treating diabetic retinopathy or other small vessel disorders associated with diabetes mellitus, a method of treating symptomatic dermographism or dermatitis, a method of treating allergic rhinitis, or a method of treating histamine-induced disorders.

In a fifth aspect of the invention, there is provided a pharmaceutical composition for the treatment of histamine-induced disorders comprising large particles of descarboethoxyloratadine, or a pharmaceutically acceptable salt thereof, and a pharmaceutically acceptable carrier, wherein the descarboethoxyloratadine, or pharmaceutically acceptable salt thereof, present in the composition has a particle size distribution in which greater than 40% by weight of the descarboethoxyloratadine, or pharmaceutically acceptable salt thereof, comprises particles having a size of 250 µm or larger.

In a sixth aspect of the invention, there is provided a solid pharmaceutical composition for the treatment of histamine-induced disorders comprising coated descarboethoxyloratadine, or a pharmaceutically acceptable salt thereof and a pharmaceutically acceptable carrier, the coated descarboethoxyloratadine, or pharmaceutically acceptable salt thereof, comprising a granulated formulation of descarboethoxyloratadine, or a pharmaceutically acceptable salt thereof, and a pharmaceutically acceptable inert excipient, wherein the granulated formulation is coated with an inert coating.

In a seventh aspect of the invention, there is provided an instant release solid pharmaceutical dosage form for treating histamine-induced disorders, comprising an open matrix network carrying descarboethoxyloratadine, or a pharmaceutically acceptable salt thereof, wherein the open matrix network comprises a pharmaceutically acceptable water-soluble or water-dispersible carrier that does not interact with the descarboethoxyloratadine, or a pharmaceutically acceptable salt thereof, said instant release dosage form being adapted to disintegrate rapidly in water.

Preferred embodiments of the invention, in any of its various aspects, are as described below or as defined in the sub-claims.

Recognizing the desirability of DCL-containing pharmaceutical compositions, we have concluded that under typical manufacturing and storage conditions, DCL is not stable and degrades in the presence of lactose, a compound commonly used as a filler in various pharmaceutical dosage forms, such as tablets, capsules or powders. Over time, the lactose and DCL compound form a brown-colored product, and there is a high degree of DCL degradation. The intensity of the brown color is typically dependent on the amount of DCL present, the conditions of storage, such as humidity and temperature, as well as the length of storage time.

Stability of a pharmaceutical product may be defined as the capability of a particular formulation, in a specific container, to remain within its physical, chemical, microbiological, therapeutic and toxicological specification, although there arc exceptions, and to maintain at least about 90% of the product's labeled potency level. Thus, for example, expiration dating is defined as the time in which the pharmaceutical product will remain stable when stored under recommended conditions.

The stability of a pharmaceutical product may be affected by several factors. including the stability of the therapeutic ingredient(s), the potential interaction between therapeutic and inactive ingredient(s) and the like. In addition, as previously indicated, physical factors such as heat, light and moisture may accelerate or initiate chemical interactions and the degradation of the product.

While not wishing to be restricted to any particular theory, it is believed that in the present case, lactose may react with DCL, degrading it to form an enamine. Such a reaction may also occur with other similar reactive excipients, such as other mono- or di-saccharides. Stable pharmaceutical compositions of DCL, or a pharmaceutically acceptable salt thereof, in blended, granulated or compressed form, which are substantially free of reactive excipients are therefore especially desirable.

The present invention relates to stable pharmaceutical compositions of DCL wherein DCL is in intimate admixture with one or more excipient(s), including, but not limited to, blended, granulated or compressed dosage forms, that avoid the incompatibility between DCL and reactive excipients, such as lactose and other mono- or di-saccharides.

Accordingly, a preferred embodiment of the present invention provides stable pharmaceutical compositions, substantially free of reactive excipients, such as lactose or other mono- or di-saccharides, in blended or granulated form, comprising descarboethoxyloratadine, or a pharmaceutically acceptable salt thereof, and one or more pharmaceutically acceptable inert excipients. In another embodiment, the present invention relates to chemically stable pharmaceutical compositions, in blended or granulated dosage forms, that are substantially free of reactive excipients, and that comprise about 1% to about 50% by weight of DCL, or a pharmaceutically acceptable salt thereof, and about 99% to about 50% by weight of at least one pharmaceutically acceptable inert excipient. The compositions provide effective, non-sedating antihistaminic activity, while avoiding the often severe adverse side-effects associated with the use of other antihistamines. Thus. the disclosed compositions arc beneficial in the treatment of numerous histamine-induced disorders including, but not limited to, allergic asthma, urticaria,

Moreover, because the disclosed compositions avoid the effects associated with other non-sedating antihistamines, an interaction between the compositions and agents that inhibit cytochrome P450 is also avoided. Such agents include, but are not limited to, ketoconazole, itraconazole and macrolides, such as erythromycin.

In addition to the active DCL ingredient, the disclosed compositions may also include a therapeutically effective amount of a non-steroidal antiinflammatory agent or other non-narcotic analgesic, such as acetylsalicylic acid, acetaminophen, ibuprofen, ketoprofen or naproxen. Such combination compositions are beneficial for the treatment of cough, cold, cold-like and/or flu symptoms and the discomfort, pain, headache, fever and general malaise associated therewith.

Other combination compositions beneficial in the treatment of these symptoms may include, in addition to an analgesic, a therapeutically effective amount of one or more other active components, such as a decongestant, *e*.*g*., pseudoephedrine, a cough suppressant/antitussive, *e.g.,* dextromethorphan, or an expectorant, *e.g.*, guaifenesin.

Interestingly, our studies have also shown that in the absence of unbound water very little to no degradation occurs in DCL compositions that include lactose. While under typical packaging and storage conditions, DCL pharmaceutical composition dosage forms would be exposed to unbound water, *e*.*g*., in the form of humidity, there arc known manufacturing and storage procedures by which exposure to unbound water and humidity is reduced or eliminated.

Moreover, although excipients other than lactose may be readily used to manufacture the disclosed pharmaceutical compositions of DCL without impacting the manufacturability and therapeutic performance of the composition, spray-dried lactose continues to be an excipient of choice. In the spray-dried form, lactose is among the best of all direct compression fillers in fluidity and is very effective for low dose formulations (<50 mg per dosage) where the compactability of the active dose does not play a major role. *See*, *e*.*g*., R. Shangraw, *Selection of Manufacturing Process and Excipients with an Emphasis on Direct Compression*, Course material from Granulation, Tableting and Capsule Technology, Center for Professional Advancement, East Brunswick, N.J. (1996). Therefore, when possible, it is desirable to include lactose among the available potential excipients for the development of solid dosage forms.

Accordingly, another embodiment of the present invention encompasses non-hygroscopic pharmaceutical compositions comprising DCL, or a pharmaceutically acceptable salt thereof, and at least one pharmaceutically acceptable excipient. Non-hygroscopic pharmaceutical compositions according to the present invention may contain excipients that are substantially free of unbound water, *i*.*e*., water which is available to participate in DCL/reactive excipient interactions, such as, but not limited to, interactions between lactose and DCL, such excipients include lactose and other reactive excipients, for example, other mono- or di-saccharides.

It should be noted that non-hygroscopic pharmaceutical compositions of the present invention may nevertheless include some hygroscopic ingredients; however, the overall composition must be substantially non-hygroscopic. Further, suitable excipients for use in such non-hygroscopic pharmaceutical compositions include hydrated excipients, such as α-lactose monohydrate and the like.

Traditionally, when pharmaceutical compositions or formulations are prepared, the active ingredient or therapeutic agent (e.g., DCL) is milled and/or screened to decrease the particle size and/or narrow the particle size distribution. Most often, this is done in order to optimize various physicochemical characteristics of the formulation, such as dissolution, content uniformity, bioavailability of the active ingredient, and the like. However, the interaction between DCL and reactive excipients, such as lactose, may be affected by the surface area of the DCL particles in the pharmaceutical composition or formulation.

Thus, another embodiment of the present invention encompasses pharmaceutical compositions for the treatment of histamine-induced disorders, comprising DCL, or a pharmaceutically acceptable salt thereof, consisting of large particles, and a pharmaceutically acceptable carrier. Pharmaceutically acceptable carriers suitable for use in these compositions may comprise one or more excipients selected from the group consisting of inert excipients and reactive excipients, such as lactose or other mono- or di-saccharides. These "large particle" pharmaceutical compositions of DCL have suitable physicochemical characteristics (in terms of dissolution, content uniformity, bioavailability, and the like), but do not exhibit incompatibility with reactive excipients, such as lactose.

In a preferred embodiment, the DCL, or a pharmaceutically acceptable salt thereof, present in the composition has a particle size distribution in which greater than about 40% by weight of DCL, or a pharmaceutically acceptable salt thereof, comprises particles having a size of 250 µm or larger.

Another means for inhibiting or preventing the interaction between DCL and reactive excipients, such as lactose, in a pharmaceutical composition is to prevent DCL from coming into contact with any reactive excipients in the composition. One manner in which this may be achieved is to coat the DCL particles with an inert or non-reactive coating prior to formulation with reactive excipients. Preferably, the inert coating should not significantly influence the pharmacodynamic characteristics (*e*.*g*., time to onset of efficacy, and adsorption *in vivo*) of the composition.

Accordingly, yet another embodiment of the present invention relates to solid pharmaceutical compositions for the treatment of histamine-induced disorders comprising a therapeutically effective amount of coated DCL, or a pharmaceutically acceptable salt thereof, which comprises DCL, or a pharmaceutically acceptable salt thereof, coated with an inert coating agent, and a pharmaceutically acceptable carrier. In a preferred embodiment, the DCL or a pharmaceutically acceptable salt thereof, is first granulated with an inert excipient, such as starch, and then the resulting granules are coated with an inert or non-reactive coating agent. Thereafter, the resulting coated DCL may be blended with other excipients, including reactive excipients.

Suitable inert coating agents, and methods for coating particles or granules, are well known in the art. Inert coating agents typically comprise an inert film-forming agent dispersed in a suitable solvent, and may further comprise other pharmaceutically acceptable adjuvants, such as colorants and plasticizers. Preferably, the particles or granules of DCL are coated using aqueous or non-aqueous film coating techniques or microencapsulation. Suitable inert film-forming agents include, but are not limited to, cellulosics, such as methylcellulose, hydroxymethyl cellulose, carboxymethylcellulose, hydroxypropyl methylcellulose, hydroxypropyl cellulose, hydroxyethylcellulose. methylhydroxyethylcellulose and sodium carboxymethyl cellulose; vinyls, such as polyvinyl pyrrolidone; glycols, such as polyethylene glycols: acrylics. such as dimethylaminoethyl methacrylate-methacrylate acid ester copolymer, and ethylacrylate-methylacrylate copolymer; and other carbohydrate polymers, such as maltodextrins. and polydextrose. Preferably, the inert coating agent contains a hydrophilic film-forming agent, such as hydroxypropyl methylcellulose, so that absorption *in vivo* is not significantly delayed.

Once the particles or granulated formulations of DCL arc coated with the inert coating agent, the coated DCL may be formulated using standard techniques, including, but not limited to, blending, granulation, compression and combinations thereof, with other inert and reactive excipients, such as lactose, to make various dosage forms, for example, tablets, caplets, capsules, troches, and the like.

DCL may also be formulated in instant release dosage forms, such as those taught in United States Patent 4,371,516 to Gregory *et al*. Instant release dosage forms of DCL may be particularly advantageous for certain uses as these dosage forms allow the DCL to be rapidly absorbed by the patient. The term "instant release" as used herein means that the dosage form or pharmaceutical composition disintegrates rapidly, *e.g*., within 10 seconds, in water. The disintegration time may be measured using procedures well known in the art, such as the procedures set forth in United States Patent 4,371,516. DCL may also be formulated in effervescent dosage forms, which may be prepared using techniques well known in the art. Effervescent dosage forms typically contain sodium bicarbonate and either citric acid, tartaric acid or sodium biphosphate in addition to the active ingredient (*e*.*g*., DCL). When mixed with water, carbon dioxide is released as a result of the acid-base reaction. It should be noted that instant release or effervescent dosage forms of DCL should not be formulated with reactive excipients, such as lactose or other mono-or di-saccharides.

Accordingly, yet another embodiment of the present invention encompasses instant release solid pharmaceutical dosage forms for treating histamine-induced disorders comprising an open matrix network carrying a therapeutically effective amount of DCL, or a pharmaceutically acceptable salt thereof, wherein the open matrix network comprises a pharmaceutically acceptable water-soluble or water-dispersible carrier that does not interact with DCL, or a pharmaceutically acceptable salt thereof.

Suitable carriers or fast dissolving inert carriers for use in the instant release pharmaceutical dosage forms of the present invention include, but are not limited to, polypeptides, such as gelatin, and in particular, hydrolyzed gelatin; polysaccharides, such as hydrolyzed dextran or dextrin; alginates, such as sodium alginate; and mixtures thereof. The carrier may also include other inert excipients, such as polyvinyl alcohol, polyvinylpyrrolidone, acacia, mannitol, sorbitol, glycine, and mixtures thereof. See, United States Patent 4,371,516. In addition, the carrier may further include pharmaceutically acceptable adjuvants, such as, for example, coloring agents, flavoring agents, preservatives, and the like.

Still another embodiment of the present invention provides anhydrous pharmaceutical compositions of descarboethoxyloratadine for the treatment of histamine-induced disorders, comprising a therapeutically effective amount of DCL, or a pharmaceutically acceptable salt thereof, and a pharmaceutically acceptable carrier or excipient. Carriers or excipients that may be used in the anhydrous compositions of the present invention include the inert excipients useful in the stable pharmaceutical compositions substantially free of reactive excipients according to the present invention, as well as lactose or other reactive excipients such as mono- or di-saccharide excipients.

Anhydrous pharmaceutical compositions should be prepared and stored in a manner that maintains an overall substantially anhydrous composition. For example, such compositions may be prepared using anhydrous or low moisture ingredients, using low moisture or humidity conditions, and the like, such that the resulting pharmaceutical compositions are substantially anhydrous, *i*.*e*., substantially free of unbound water.

In addition, as with the disclosed pharmaceutical compositions, in blended, granulated or compressed form, that arc substantially free of reactive excipients, the non-hygroscopic and anhydrous pharmaceutical compositions of DCL may include a therapeutically effective amount of a non-steroidal antiinflammatory agent or other non-narcotic analgesic, as well as a therapeutically effective amount of one or more other active components, such as a decongestant, an antitussive, or an expectorant. Examples of such therapeutic agents include all of those available for the DCL compositions substantially free of reactive excipients, such as lactose.

Anhydrous DCL pharmaceutical compositions prepared in accordance with the present invention should be prepared and stored such that the anhydrous nature is maintained. Accordingly, these compositions will be packaged using materials known in the art for preventing exposure of the composition to water, allowing them to be included in suitable formulary kits. Such packaging will include, but not be restricted to, hermetically sealed foil, plastic or the like, and unit dose containers, *e*.*g*., blister packs or strip packs. These forms of packaging may also be used with any of the other dosage forms disclosed herein.

As mentioned above, pharmaceutical compositions or formulations of DCL containing lactose, or other reactive excipients, that are exposed to unbound water, *e.g*., moisture or humidity, degrade more rapidly. The addition of water (e.g., 5%) is widely accepted in the pharmaceutical arts as a means of simulating long-term storage in order to determine characteristics such as shelf-life or the stability of formulations over time. *See*, *e*.*g*., Jens T. Cartensen, *Drug Stability: Principles & Practice*, 2d. Ed., Marcel Dekker, NY, NY, 1995, pp. 379-80. In effect, water and temperature accelerate the study.

Further, the effect of water on a formulation is of great significance since conditions favorable for hygroscopicity, *e*.*g*., moisture and/or humidity, are commonly encountered during manufacture, handling, packaging, storage, shipment and use of the formulation. Thus, it is clear that the use of lactose, or other reactive excipients such as other mono-or di-saccharide excipients, in pharmaceutical compositions or formulations containing DCL should be avoided due to the substantial contact with moisture and/or humidity that the compositions experience under normal manufacturing, packaging and storage conditions.

Stability of a pharmaceutical product or composition may be defined as the capability of a particular formulation, in a specific container, to remain within its physical, chemical, microbiological, therapeutic and toxicological specification, although there are exceptions, and to maintain at least about 90% of its labeled potency level. Thus, expiration dating, for example, is defined as the time for which the pharmaceutical product or composition will remain stable when stored under recommended conditions.

Many factors affect the stability of a pharmaceutical product or composition, and include, for example, the stability of the therapeutic ingredient(s), the potential interaction or incompatibility between therapeutic and inactive ingredient(s) (*e*.*g*., the interaction between DCL and certain excipients, such as lactose) and the like.

DCL degradation does not occur in the presence of other non-reactive excipients. The terms "inert excipient(s)" and "non-reactive excipient(s)" as used herein are intended to mean excipients, including, but not limited to, binders/fillers, disintegrants, lubricants, anti-caking agents, dispersing agents, preservatives, film coating agents, plasticizers, surface active agents, and the like, which are compatible with and do not interact with DCL under typical manufacturing, packaging and storage conditions. Inert excipients or non-reactive excipients which may be used in the present invention are well known in the art, and include, but are not limited to, maltodextrin, cellulose, calcium phosphate, calcium phosphate dihydrate, calcium carbonate, talc, calcium stearate, calcium sulfate dihydrate and corn starch. Furthermore, inert or non-reactive excipients provide a pharmaceutical composition that is comparable in manufacturability and therapeutic performance as those utilizing lactose.

The term "inert carrier" as used herein refers to a carrier or vchicle comprising one or more inert excipients or non-reactive excipients.

As used herein, the term "reactive excipient(s)" refers to excipients that react with DCL in the presence of unbound water, and include, for example, lactose and other mono-or di-saccharide excipients. The terms "substantially free of reactive excipient(s)", "substantially free of lactose" and "lactose-free" as used herein arc intended to mean that the amount of reactive excipient(s), or lactose as appropriate, present, if any, in the dosage form or pharmaceutical composition of DCL is insufficient to cause the incompatibility between DCL and the particular excipient(s), such as lactose, discovered by the present inventors to detrimentally affect the potency of the DCL below about 90% of its initial potency over the shelf life of the dosage form or pharmaceutical composition. See. standards set forth in the USP XXI/NF XVI. Typically, the amount of any reactive excipients that may be present in compositions of the present invention that are substantially free of reactive substituents should be less than about 20% by weight, preferably less than about 10% by weight, and even more preferably, less than about 1 % by weight.

The term "unbound water" as used herein refers to water that is not present in the form of a stable hydrate of one or more components of the pharmaceutical composition. *e*.*g*., α-lactose monohydrate. Similarly, the term "anhydrous" as used herein means that the amount of unbound water present, if any, in the dosage form or pharmaceutical composition of DCL is insufficient to initiate and/or accelerate the incompatibility between DCL and reactive excipients, such as lactose. Further, "anhydrous conditions" or nature as used herein means substantially free of unbound water, including moisture. The term "non-hygroscopic" as used herein means the overall formulation or pharmaceutical composition is substantially non-hygroscopic, *i.e.,* it does not provide unbound water sufficient to initiate and/or accelerate the incompatibility between DCL and reactive excipients, such as lactose.

The term "substantially free of unbound water" typically means that less than about 5 weight percent, preferably less than about 1 weight percent, and more preferably, less than about 0.1 weight percent, of water is present.

DCL may be present in pharmaceutical compositions prepared in accordance with the present invention as either a free base or as a pharmaceutically acceptable salt thereof. "Pharmaceutically acceptable salt" refers to a salt prepared from pharmaceutically acceptable non-toxic organic or inorganic, acids or bases. Examples of such organic acids include, for example, aliphatic, aromatic, carboxylic and sulfonic classes of organic acids, such as formic, acetic, propionic, succinic, glycolic, glutamic, glucouronic, maleic, furoic, benzoic, anthranilic, salicylic, phenylacetic, mandelic, embonic (pamoic), methanesulfonic, ethanesulfonic. benzenesulfonic, pantothenic, stearic, sulfanilic, galacturonic and algenic. Examples of such inorganic acids arc hydrochloric, hydrobromic, hydroiodic, sulfuric and phosphoric. Examples of such organic bases include, for example, N,N-dibenzylethylenediamine, chloroprocaine, choline, diethanolamine, ethylenediamine, meglumaine (N-methylglucamine), lysine and procaine. Examples of such inorganic bases include metallic salts made from lithium, aluminum, calcium, magnesium, potassium, sodium and zinc.

Pharmaceutical compositions of the present invention may also comprise (i) a therapeutically effective amount of DCL or a pharmaceutically acceptable salt thereof, and (ii) a therapeutically affective amount of at least one non-steroidal antiinflammatory agent or non-narcotic analgesic or a pharmaceutically acceptable salt thereof.

In addition, the disclosed compositions may comprise (i) a therapeutically effective amount of DCL or a pharmaceutically acceptable salt thereof, and (ii) a therapeutically affective amount of a decongestant or a pharmaceutically acceptable salt thereof.

"Therapeutically effective amount" means that amount, in the case of DCL, or a pharmaceutically salt thereof, which provides a therapeutic benefit in the treatment and management of histamine-induced disorders, including but not limited to, allergic rhinitis and other allergic disorders such as urticaria, symptomatic dermographism, dermatitis, allergic asthma, diabetic retinopathy or other small vessel disorders associated with diabetes mellitus and the symptoms associated with allergic rhinitis such as cough, cold, cold-like and/or flu symptoms including but not limited to, sneezing, rhinorrhea, lacrimation and dermal irritation.

The magnitude of a prophylactic or therapeutic dose of DCL in the acute or chronic management of disease will vary with the severity of the condition to be treated. The dose, and perhaps the dose frequency, will also vary according to the age, body weight and response of the individual patient. In general, the total daily dose range, for the conditions described herein, is from about 0.1 mg to about 10 mg, administered in single or divided doses. Preferably, an oral daily dose range is from about 0.1 mg to about 5 mg, and more preferably, from about 0.2 mg to about 1 mg.

It is further recommended that children, patients over 65 years of age, and those with impaired renal or hepatic function, initially receive a lower dose, and that they then be titrated based upon individual response or blood lever. As will be apparent to those skilled in the art, it may be necessary to use dosages outside these ranges in particular cases. It is further noted that the clinician or treating physician will know how and when to interrupt, adjust or terminate a dosage regimen based upon individual patient response.

"Therapeutically effective amount of DCL or a pharmaceutically acceptable salt thereof" is encompassed within the above-described dosages. In addition, the phrases "comprising (i) a therapeutically effective amount of DCL or a pharmaceutically acceptable salt thereof, and (ii) a therapeutically affective amount of at least one non-steroidal antiinflammatory agent or non-narcotic analgesic or a pharmaceutically acceptable salt thereof" and "comprising (i) a therapeutically effective amount of DCL or a pharmaceutically acceptable salt thereof, and (ii) a therapeutically affective amount of a decongestant or a pharmaceutically acceptable salt thereof" are also encompassed by the above-described dosages and dose frequency schedules.

Pharmaceutical compositions of the present invention may be administered by any suitable route of administration that provides a patient with a therapeutically effective dosage of DCL. Typically, the DCL pharmaceutical compositions described herein will be formulated for oral administration. Suitable dosage forms include tablets, troches, cachets, caplets, capsules, including hard and soft gelatin capsules, and the like. Tablet forms, however, remain a preferred dosage form because of advantages afforded both the patient (*e.g*., accuracy of dosage, compactness, portability, blandness of taste and ease of administration) and to the manufacturer (*e*.*g*., simplicity and economy of preparation, stability and convenience in packaging, shipping and dispensing).

The pharmaceutical compositions substantially free of reactive excipients may further include a "pharmaceutically acceptable inert carrier" and this expression is intended to include one or more inert excipients, which include starches, polyols, granulating agents, microcrystalline cellulose, diluents, lubricants, binders, disintegrating agents, and the like, other than lactose. The anhydrous, non-hygroscopic, and other compositions according to the present invention may include any "pharmaceutically acceptable carrier", and this expression is intended to include one or more inert excipients, as well as reactive excipients, such as α-lactose monohydrate. If desired, tablet dosages of the disclosed compositions may be coated by standard aqueous or nonaqueous techniques, with the proviso that nonaqueous coatings and coating techniques should be used for tablets of disclosed compositions that are not substantially free of reactive excipients. "Pharmaceutically acceptable carrier" also encompasses controlled release means.

Compositions of the present invention may also optionally include other therapeutic ingredients, anti-caking agents, preservatives, sweetening agents, colorants, flavors, desiccants, plasticizers, dyes, and the like. However, any such optional ingredient must be compatible with DCL to insure the stability of the formulation.

Examples of excipients for use as the pharmaceutically acceptable carriers and the pharmaceutically acceptable inert carriers and the aforementioned additional ingredients include. but are not limited to:
BINDERS: corn starch, potato starch, other starches, gelatin, natural and synthetic gums such as acacia, sodium alginate. alginic acid, other alginates. powdered tragacanth, guar gum, cellulose and its derivatives (e.g., ethyl cellulose, cellulose acetate. carboxymethyl cellulose calcium, sodium carboxymethyl cellulose), polyvinyl pyrrolidone. methyl cellulose, pre-gelatinized starch (*e*.*g*., STARCH 1500® and STARCH 1500 LM®, sold by Colorcon, Ltd.), hydroxypropyl methyl cellulose, microcrystalline cellulose (e.g. AVICEL™, such as, AVICEL-PH-101™, -103™ and -105™, sold by FMC Corporation, Marcus Hook, PA, USA), or mixtures thereof;
FILLERS: talc, calcium carbonate (e.g., granules or powder), dibasic calcium phosphate, tribasic calcium phosphate, calcium sulfate (e.g., granules or powder), microcrystalline cellulose, powdered cellulose, dextrates, kaolin, mannitol, silicic acid, sorbitol, starch, pre-gelatinized starch, or mixtures thereof;
DISINTEGRANTS: agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, clays, other algins, other celluloses, gums, or mixtures thereof;
LUBRICANTS: calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil *e*.*g*., peanut oil, cottonseed oil, sunflower oil, sesame oil, olive oil, corn oil and soybean oil), zinc stearate, ethyl oleate, ethyl laurate, agar, syloid silica gel (AEROSIL 200, W.R. Grace Co., Baltimore, MD USA), a coagulated aerosol of synthetic silica (Deaussa Co., Piano, TX USA), a pyrogenic silicon dioxide (CAB-O-SIL, Cabot Co., Boston, MA USA), or mixtures thereof;
ANTI-CAKING AGENTS: calcium silicate, magnesium silicate, silicon dioxide, colloidal silicon dioxide, talc, or mixtures thereof;
ANTIMICROBIAL AGENTS: benzalkonium chloride, benzethonium chloride, benzoic acid, benzyl alcohol, butyl paraben, cetylpyridinium chloride, cresol, chlorobutanol, dchydroacetic acid, ethylparaben, methylparaben, phenol, phenylethyl alcohol, phenylmercuric acetate, phenylmercuric nitrate, potassium sorbate, propylparaben, sodium benzoate. sodium dehydroacetate, sodium propionate, sorbic acid, thimersol, thymo, or mixtures thereof; and
COATING AGENTS: sodium carboxymethyl cellulose, cellulose acetate phthalate, ethylcellulose, gelatin, pharmaceutical glaze, hydroxypropyl cellulose, hydroxypropyl methylcellulose, hydroxypropyl methyl cellulose phthalate, methylcellulose, polyethylene glycol, polyvinyl acetate phthalate, shellac, sucrose, titanium dioxide, carnuba wax, microcrystalline wax, or mixtures thereof.

The disclosed compositions may be prepared to include any of the mentioned ingredients, by any of the methods of pharmacy, with the proviso that the substantially free of reactive excipients, non-hygroscopic or anhydrous nature of a given composition is maintained. Tablets, for example, may be prepared by compression or molding, optionally, with one or more accessory ingredients, consistent with the nature of the particular composition and with the principles of the present invention. Compressed tablets may be prepared by compressing in a suitable machine the active ingredient in a free-flowing form such as powder or granules, optionally mixed with a binder, lubricant, inert diluent, surface active or dispersing agent or other compatible excipient as described herein above. Desirably, each tablet contains from about 0.1 mg to about 10 mg, and more desirably, from about 0.1 to about 5 mg, of the active ingredient, DCL or a pharmaceutically acceptable salt thereof.

It is noted that all components comprising the dosage forms of DCL made in accordance with any of the embodiments of the present invention preferably meet or exceed the standards for pharmaceutical ingredients and combinations thereof in the USP/NF. The purpose of the USP/NF is to provide authoritative standards and specifications for materials and substances and their preparations that are used in the practice of the healing arts. The USP/NF establishes titles, definitions, descriptions and standards for the identification, quality, strength, purity, packaging and labeling of such materials and substances. Also, where practicable, provides handling and storage information, formulas for their manufacture and preparation and methods for their examination.

The compositions of DCL described and claimed herein meet the pharmaceutical standards set forth in the USP/NF (*e.g*., USP XXI/NF XVI) for each of the ingredients and for each of the various dosage forms made with such ingredients. In effect, the disclosed compositions of DCL are said to be pharmaceutically acceptable dosage forms made of pharmaceutically acceptable ingredients in pharmaceutically acceptable combinations and pharmaceutically acceptable amounts to at least meet the standards set forth in the USP XXI/NF XVI.
I. A first study was carried out to determine the chemical compatibilities of DCL with common excipients by differential scanning calorinictry (DSC). The study used dcscarboethoxyloratadine (Lot 589-YF-15A) as the active drug.
Excipients tested:
AVICEL™ microcrystalline cellulose
STARCH 1500®
Lactose (α-lactose monohydrate)

Procedure: The various excipients were dry blended with DCL (80% excipient to 20% drug). DSC runs were performed on each blend as well as neat drug substance.
Results: The DSC curve for DCL exhibits an endothermic melt peak at 149.82° C. When dry blended with lactose, the low melting endotherm for lactose and the melting endotherm for DCL combine to produce a single melt indicative of a solid dispersion. The higher melting endotherm for lactose was also reduced to a lower temperature. This behavior is indicative of an active drug/excipient interaction.
The DSC trace for a dry blend of AVICEL™ and DCL shows one endotherm at 147.55° C, the approximate melting point of DCL in the absence of AVICEL™. Since the baseline is affected adversely by the presence of AVICEL™, the slight difference in melting point can be attributed to error in the extrapolated onset temperature. Therefore, the endotherm for the blend appears to be the same as for neat DCL and no interaction with AVICEL™ is apparent.
As for the AVICEL™ blend, the dry blending of STARCH 1500® and DCL produce one peak at 147.75° C. Thus. there does not appear to be a DCL/STARCI 1500® interaction. These results are presented in Table 1, below.

**Table 1**

| SUBSTANCE | COMMENTS |
|---|---|
| DCL | one peak 149.82°C |
| AVICEL™ | no peak (does not melt) |
| STARCH 1500® | no peak (does not melt) |
| α-LACTOSE monohydrate | two peaks 140.81°C, 210.47°C |
| DCL/AVICEL™ | one peak 147.55°C - no interaction |
| DCL/STARCH 1500® | one peak 147.75°C - no interaction |
| DCL/α-LACTOSE monohydrate | two peaks 145.04°C. 195.17°C solid dispersion |

II. A second study was carried out to determine the stability of a formulation comprised of DCL and lactose. in the presence and absence of 5% water. The study used descarboethoxyloratadine (Lot 589-YF-15A).
Procedure: A series of amber 20 ml crimp-topped vials were prepared to contain DCL and lactose. The contents of the vials were (1) dry DCL; (2) 20% dry DCL and 80% lactose; and (3) 20% DCL, 80% lactose and 5% H₂O. The vials were placed in a 60° C oven for 16 days and then assayed via high-performance liquid chromatography (HPLC) at 256 nanometers.
Results: The only significant degradation seen was in the vial containing 5% H₂O. This sample represents the worst case scenario for a drug/excipient interaction as stated in *Drug Stability* (Carstensen *et al*., pp. 379-380). Thus, these data indicate that under common accelerated conditions for excipient interaction studies, the presence of α-lactose monohydrate has been shown to adversely affect the stability of DCL, while a solid dose DCL/lactose composition in the absence of 5% moisture does not show this high degree of degradation. These results are presented in Table 2, below.

**Table 2**

| SAMPLE | T₀ wt. DCL (mg) | T₁₆ wt. DCL (mg) | %DEGRADATION |
|---|---|---|---|
| DCL | 28.70 | 28.70 | 0 |
| DCL/LACTOSE | 19.82 | 19.74 | 0.40 |
| DCL/LACTOSE/5%H₂O | 39.70 | 19.51 | 50.86 |

III. A third study was carried out to evaluate the reactivity of DCL with lactose, in the presence and absence of intentionally introduced water. The effects of reduced particle size were also evaluated by comminuting blends of DCL/lactose in a mortar and pestle prior to storage. Samples were stored in crimp-topped vials (as above), under accelerated conditions (60° C at 75% Relative Humidity) for various time periods and then tested for reactivity of DCL with lactose. The results are shown in Table 3, below.

**Table 3 Reactivity of DCL with Lactose**

| Storage Time (60° C / 75% RH) | Treatment | % Initial |
|---|---|---|
| 4 weeks | neat | 99.70 |
| 4 weeks | 80% lactose | 68.58 |
| 4 weeks | 80% lactose / 5% H₂O | 49.57 |
| 1 week | 80% lactose / 5% H₂O* | 90.35 |
| 2 weeks | 80% lactose / 5% H₂O* | 49.07 |
| 4 weeks | 80% lactose / 5% H₂O* | 48.80 |
| 4 weeks | 80% lactose ** | 46.95 |
| 4 weeks | 80% lactose ** | 49.52 |

| | | |
|---|---|---|
| * DCL and lactose particle sizes reduced with mortar and pestle | | |
| ** High surface area Fast Flo® lactose | | |

As can be seen from the results, the reaction rate and/or the extent of DCL/lactose interaction, is reduced in the absence of added water. In addition, a reduction in the particle size of DCL and lactose led to the same extent of reaction after 2 and 4 weeks of storage under accelerated conditions. It is not possible to determine if this rate was accelerated relative to the material that was not reduced in size because of the lack of comparative data. It is noteworthy, however, that samples containing high surface area lactose led to reaction even in the absence of added water. This result indicates that the reaction rate is surface area dependent beyond some threshhold value, as Fast Flo® lactose, despite its desirable flow and compressability characteristics, led to a faster degradation rate.

As shown in Table 4, below, the reactivity of loratadine with lactose was negligible under similar conditions, including samples where 5% water was introduced .

**Table 4 Reactivity of Loratadine with Lactose**

| Storage Time (60° C / 75% RH) | Treatment | % Initial |
|---|---|---|
| 4 weeks | neat | 99.35 |
| 4 weeks | 80% lactose | 100.33 |
| 4 weeks | 80% lactose / 5% H₂O | 100.37 |

### EXAMPLES

Various embodiments of the present invention are hereinafter described in more detail by means of the following examples of pharmaceutical compositions of DCL, substantially free of reactive excipients, that arc provided by way of illustration and not by way of limitation.

### Example 1

Compressed DCL tablets may be prepared using conventional wet granulation techniques, such that each dosage unit contains 0.1 mg to 10 mg of DCL.

| | Per tablet | Per 10,000 tablets |
|---|---|---|
| DCL | 10 mg | 100 g |
| Starch | 60 mg | 600 g |
| Talc | 12 mg | 120 g |
| Acacia | 12 mg | 120 g |
| Stearic Acid | 1 mg | 10 g |

The acacia and an equal weight of starch is blended to form a paste which is used to granulate the DCL. The mixture is dried and placed through a mesh screen. The remainder of the material is added and mixed thoroughly. The resulting mixture is compressed into tablets using a 9/32-inch (7 mm) punch.

### Example 2

Compressed DCL tablets may be prepared using conventional dry granulation techniques, such that each dosage unit contains 0.1 mg to 10 mg of DCL.

| | Per tablet | Per 10.000 tablets |
|---|---|---|
| DCL | 10 mg | 100 g |
| Starch | 85 mg | 850 g |

The starch is dried to a moisture content of 10% and then thoroughly mixed with the DCL. The resulting mixture is compressed into slugs and then ground to fine mesh size. Tablets arc then compressed, using a 9/32-inch (7mm) punch.

### Example 3

Compressed DCL tablets may be prepared using conventional direct compression techniques, such that each dosage unit contains 0.1 mg to 10 mg of DCL.

| | Per tablet | Per 10.000 tablets |
|---|---|---|
| DCL. | 10 mg | 100g |
| Microcrystalline Cellulose | 80 mg | 800 g |
| Stearic Acid | 5 mg | 50 g |
| Colloidal Silica | 1 mg | 10 g |

All of the ingredients are blended in a suitable blender. The resulting mixture is compressed into tablets, using a 9/32-inch (7mm) punch.

### Example 4

Chewable DCL tablets may also be prepared using conventional direct compression techniques, such that each dosage unit contains 0.1 mg to 10 mg of DCL.

| | Per tablet | Per 10.000 tablets |
|---|---|---|
| DCL | 10 mg | 100 g |
| Mannitol, USP | 70 mg | 700 g |
| Microcrystalline Cellulose | 7 mg | 70 g |
| Corn starch | 3 mg | 30 g |
| Calcium stearate | 2 mg | 20 g |
| Flavoring agent | qs | qs |

All of the ingredients are blended in a suitable blender. The mixture is compressed into tablets using a 9/32-inch (7mm) flat-face bevel-edge punch.

### Example 5

Soft gelatin DCL capsules may be prepared with a mixture of DCL in a digestible oil such as soybean oil, lecithin, cottonseed oil, or olive oil wherein the mixture is injected by means of a positive pressure pump into gelatin, such that each dosage unit contains 0.1 mg to 10 mg of DCL. The capsules are washed and dried.

### Example 6

This example is provided as an illustration of an anhydrous pharmaceutical composition of DCL that includes lactose. Compressed DCL tablets may be prepared using conventional dry granulation techniques, such that each dosage unit contains 0.1 mg to 10 mg of DCL.

| | Per tablet | Per 10,000 tablets |
|---|---|---|
| DCL | 10 mg | 100 g |
| Lactose (granular, 12-mesh (1680 µm)) | 35 mg | 350 g |
| Starch | 25 mg | 250 g |
| Talc | 25 mg | 250 g |
| Magnesium stearate | 0.2 mg | 2g |

All of the ingredients are mixed thoroughly and then compressed into slugs. The slugs are then ground and screened to 14- to 16-mesh (1410 - 1190 µm) granules, which are then compressed into tablets, using a 9/32-inch (7mm) concave punch.

Tablets and capsules of other strengths may be prepared by altering the ratio of active ingredient to the excipients or to the final weight of the tablet.

## Claims

1. A pharmaceutical composition for the treatment of histamine-induced disorders, comprising 1% to 50% by weight of descarboethoxyloratadine, or a pharmaceutically acceptable salt thereof, and 99% to 50% by weight of a pharmaceutically acceptable inert carrier, wherein the composition contains lactose in an amount of less than 10% by weight and wherein the composition is in tablet form comprising descarboethoxyloratadine, or a pharmaceutically acceptable salt thereof, that has been blended or granulated with a pharmaceutically acceptable inert carrier.

2. A pharmaceutical composition as claimed in claim 1, further comprising a tablet coating.

3. A pharmaceutical composition as claimed in claims 1 or 2 comprising 0.1 mg to 10 mg of descarboethoxyloratadine.

4. A pharmaceutical composition as claimed in claim 3 comprising 0.1 mg to 5 mg of descarboethoxyloratadine.

5. A pharmaceutical composition as claimed in any one of the preceding claims further comprising an analgesic.

6. A pharmaceutical composition as claimed in claim 5, wherein the analgesic is selected from the group consisting of acetylsalicylic acid, acetaminophen, ibuprofen, ketoprofen, naproxen or a pharmaceutically acceptable salt thereof.

7. A pharmaceutical composition as claimed in any one of the preceding claims further comprising a decongestant.

8. Use of descarboethoxyloratadine, or a pharmaceutically acceptable salt thereof for the preparation of a medicament as claimed in claims 1-7 for use in a method of treating cough, cold, cold-like and flu symptoms and the discomfort, headache, pain, fever and general malaise associated therewith.

9. Use of descarboethoxyloratadine, or a pharmaceutically acceptable salt thereof for the preparation of a medicament as claimed in claims 1-7 for use in a method of treating diabetic retinopathy or other small vessel disorders associated with diabetes mellitus.

10. Use of descarboethoxyloratadine, or a pharmaceutically acceptable salt thereof for the preparation of a medicament as claimed in claims 1-7 for use in a method of treating symptomatic dermographism or dermatitis.

11. Use of descarboethoxyloratadine, or a pharmaceutically acceptable salt thereof for the preparation of a medicament as claimed in claims 1-7 for use in a method of treating allergic rhinitis.

12. Use of descarboethoxyloratadine, or a pharmaceutically acceptable salt thereof for the preparation of a medicament as claimed in claims 1-7 for use in a method of treating histamine-induced disorders.

13. A pharmaceutical composition for the treatment of histamine-induced disorders comprising large particles of descarboethoxyloratadine, or a pharmaceutically acceptable salt thereof, and a pharmaceutically acceptable carrier, wherein said particles are not incompatible with reactive excipients, including lactose, and wherein the descarboethoxyloratadine, or pharmaceutically acceptable salt thereof, present in the composition has a particle size distribution in which greater than 40% by weight of the descarboethoxyloratadine, or pharmaceutically acceptable salt thereof, comprises particles having a size of 250 µm or larger.

14. A solid pharmaceutical composition for the treatment of histamine-induced disorders comprising descarboethoxyloratadine, or a pharmaceutically acceptable salt thereof and a pharmaceutically acceptable carrier, wherein the descarboethoxyloratadine or pharmaceutically acceptable salt thereof is granulated with a pharmaceutically acceptable inert excipient and the resultant granules are coated with an inert or non-reactive coating prior to formulation with further excipients, which may include reactive excipients, including lactose.

15. The solid pharmaceutical composition of claim 14, wherein the inert coating agent comprises an inert film-forming agent in a solvent.

16. The solid pharmaceutical composition of claim 15, wherein the inert film-forming agent is selected from the group consisting of methylcellulose, hydroxymethylcellulose, carboxymethylcellulose, hydroxypropylmethylcellulose, hydroxypropylcellulose, hydroxyethylcellulose, methylhydroxyethylcellulose or sodium carboxymethylcellulose.

17. An instant release solid pharmaceutical dosage form for treating histamine-induced disorders, comprising an open matrix network carrying descarboethoxyloratadine, or a pharmaceutically acceptable salt thereof, wherein the open matrix network comprises a pharmaceutically acceptable water-soluble or water-dispersible carrier selected from the group consisting of gelatin, hydrolysed gelatin, hydrolysed dextran, dextrin, alginates, and mixtures thereof, wherein said instant release dosage form does not contain lactose and is adapted to disintegrate rapidly in water.

18. A pharmaceutical composition in blended or granulated form for the treatment of histamine-induced disorders, comprising a therapeutically effective amount of descarboethoxyloratadine, or a pharmaceutically acceptable salt thereof, and a pharmaceutically acceptable inert carrier.

19. The pharmaceutical composition of claim 18 wherein the pharmaceutical composition is substantially free of reactive excipients.

20. The pharmaceutical composition of claim 19 wherein the pharmaceutical composition is substantially free of lactose.

21. The pharmaceutical composition of claim 18 wherein the therapeutically effective amount of descarboethoxyloratadine is about 0.1 mg to 10 mg.

22. The pharmaceutical composition of claim 21 wherein the therapeutically effective amount of descarboethoxyloratadine is about 0.1 mg to 5 mg.

23. The pharmaceutical composition of claim 18 further comprising a therapeutically effective amount of an analgesic.

24. The pharmaceutical composition of claim 23 wherein the analgesic is selected from the group consisting of acetylsalicylic acid, acetaminophen, ibuprofen, ketoprofen, naproxen or a pharmaceutically acceptable salt thereof.

25. The pharmaceutical composition of claim 18 further comprising a therapeutically effective amount of a decongestant.

26. The pharmaceutical composition of claim 18 wherein the composition is present in one of tablet or capsule form.

27. The pharmaceutical composition of claim 24 wherein the composition is present in tablet form.

28. A method of treating cough, cold, cold-like and flu symptoms and the discomfort, headache, pain, fever and general malaise associated therewith, comprising administering a pharmaceutical composition according to claim 18.

29. A method of treating diabetic retinopathy or other small vessel disorders associated with diabetes melitis, comprising administering a pharmaceutical composition according to claim 18.

30. A method of treating symptomatic dermographism or dermatitis, comprising administering a pharmaceutical composition according to claim 18.

31. A method of treating allergic rhinitis, comprising administering a pharmaceutical composition according to claim 18.

32. A method of treating histamine-induced disorders, comprising administering a pharmaceutical composition according to claim 18.

33. An anhydrous pharmaceutical composition for the treatment of histamine-induced disorders, comprising a therapeutically effective amount of descarboethoxyloratadine, or a pharmaceutically acceptable salt thereof, and a pharmaceutically acceptable carrier.

34. The anhydrous pharmaceutical composition of claim 33 wherein the therapeutically effective amount of descarboethoxyloratadine is about 0.1 mg to 10 mg.

35. The anhydrous pharmaceutical composition of claim 34 wherein the therapeutically effective amount of descarboethoxyloratadine is about 0.1 mg to 5 mg.

36. The anhydrous pharmaceutical composition of claim 33 further comprising a therapeutically effective amount of an analgesic.

37. The anhydrous pharmaceutical composition of claim 36 wherein the analgesic is selected from the group consisting of acetylsalicylic acid, acetaminophen, ibuprofen, ketoprofen, naproxen or a pharmaceutically acceptable salt thereof.

38. The anhydrous pharmaceutical composition of claim 33 further comprising a therapeutically effective amount of a decongestant.

39. The anhydrous pharmaceutical composition of claim 33 wherein the composition is present in one of tablet or capsule form.

40. The anhydrous pharmaceutical composition of claim 39 wherein the composition is present in tablet form.

41. A method of treating cough, cold, cold-like and flu symptoms and the discomfort, headache, pain, fever and general malaise associated therewith, comprising administering an anhydrous pharmaceutical composition according to claim 33.

42. A method of treating diabetic retinopathy or other small vessel disorders associated with diabetes melitis, comprising administering an anhydrous pharmaceutical composition according to claim 33.

43. A method of treating symptomatic dermographism or dermatitis,comprising administering an anhydrous pharmaceutical composition according to claim 33.

44. A method of treating allergic rhinitis, comprising administering an anhydrous pharmaceutical composition according to claim 33.

45. A method of treating histamine-induced disorders, comprising administering an anhydrous pharmaceutical composition according to claim 33.

46. A non-hygroscopic pharmaceutical composition comprising descarboethoxyloratadine, or a pharmaceutically acceptable salt thereof, lactose and one or more pharmaceutically acceptable inert excipients wherein the composition is substantially free of unbound water.

47. The non-hygroscopic pharmaceutical composition of claim 46 wherein the one or more pharmaceutically acceptable inert excipients is selected from the group consisting of non-hygroscopic excipients and low-moisture excipients.

48. A solid, non-hygroscopic pharmaceutical composition comprising descarboethoxyloratadine, or a pharmaceutically acceptable salt thereof, and a pharmaceutically acceptable carrier.

49. An uncoated pharmaceutical composition substantially free of reactive excipients comprising descarboethoxyloratadine, or a pharmaceutically acceptable salt thereof, and a pharmaceutically acceptable carrier.

50. A chemically stable pharmaceutical composition in blended or granulated dosage form and substantially free of reactive excipients comprising about 1 % to about 50% by weight of descarboethoxyloratadine, or a pharmaceutically acceptable salt thereof, and about 99% to about 50% by weight of a pharmaceutically acceptable inert carrier.

51. A pharmaceutical composition for the treatment of histamine-induced disorders comprising large particles of descarboethoxyloratadine, or a pharmaceutically acceptable salt thereof, and a pharmaceutically acceptable carrier.

52. The pharmaceutical composition of claim 51 wherein the descarboethoxyloratadine, or a pharmaceutically acceptable salt thereof, present in the composition has a particle size distribution in which greater than about 40% by weight of the descarboethoxyloratadine, or a pharmaceutically acceptable salt thereof, comprises particles having a size of 250 µm or larger.

53. A solid pharmaceutical composition for the treatment of histamine-induced disorders comprising a therapeutically effective amount of coated descarboethoxyloratadine, or a pharmaceutically acceptable salt thereof, which comprises descarboethoxyloratadine, or a pharmaceutically acceptable salt thereof, coated with an inert coating agent, and a pharmaceutically acceptable carrier.

54. The solid phannaceuticat composition of claim 53 Wherein the coated descarboetboxyloratadine, or a pharmaceutically acceptable salt thereof, further comprises a granulated formulation of descarboethoxyloratadine, or a pharmaceutically acceptable salt thereof, and a pharmaceutically acceptable inert excipient, wherein the granulated formulation is coated with an inert coating agent.

55. The solid pharmaceutical composition of claim 53 or 54 wherein the inert coating agent comprises an inert film-forming agent in a solvent.

56. The solid pharmaceutical composition of claim 55 wherein the inert film-forming agent is selected from the group consisting of methylcellulose, hydroxymethyl cellulose, carboxymethylcellulose, hydroxypropylmethylcellulose, hydroxypropyl cellulose, hydroxyethylcellulose, methylhydroxyethylcellulose and sodium 5 carboxymethylcellulose.

57. An instant release solid pharmaceutical dosage form for treating histamine-induced disorders, comprising an open matrix network carrying a therapeutically effective amount of descarboethoxyloratadine, or a pharmaceutically acceptable salt thereof, wherein the open matrix network comprises a pharmaceutically acceptable water-soluble or water-dispersible carrier that does not interact with the descarboethoxyloratadine, or a pharmaceutically acceptable salt thereof.
